# EUROPEAN PATENT APPLICATION

(11) **EP 3 712 616 A1**
(43) Date of publication of application: **23.09.2020**
(21) Application number: 18879904.3
(22) Date of filing: 13.11.2018
(51) Int. Cl.: G01N 33/53, G01N 33/15, G01N 33/50, G01N 33/68

(54) **AGING CONDITION EVALUATION METHOD, INFORMATION PRESENTATION METHOD, AND SCREENING METHOD FOR SUBSTANCE THAT AMELIORATES OR PREVENTS AGING CONDITION**

(30) Priority: 14.11.2017 JP 2017218704
(71) Applicant: Hiroshima University, Higashihiroshima-shi, Hiroshima 739-8511 (JP)
(72) Inventor: KAN, Kaen, Hiroshima-shi Hiroshima 734-8553 (JP); NISHIYAMA, Yukie, Hiroshima-shi Hiroshima 734-8553 (JP); IKUO, Mariko, Hiroshima-shi Hiroshima 734-8553 (JP); TAHARA, Hidetoshi, Hiroshima-shi Hiroshima 734-8553 (JP)
(74) Representative: Haseltine Lake Kempner LLP
(86) International application number: PCT/JP2018/041990
(87) International publication number: WO 2019/098194

(57) **Abstract**

Provided are a novel method of evaluating an aging condition, a method of presenting information, and a method of screening for a substance capable of improving or preventing an aging condition. A method of evaluating an aging condition of a subject comprises the step of: comparing a value from the subject of either of the amount of extracellular vesicles including exosomes in serum, the amount of advanced glycation end products of surface proteins on the extracellular vesicles, or parameters based on the amounts with a correlation criterion, the correlation criterion with the aging condition being pre-determined.

## Description

### TECHNICAL FIELD

The present invention relates to a method of evaluating an aging condition, a method of presenting information, and a method of screening for a substance capable of improving or preventing an aging condition.

### BACKGROUND ART

Organisms show decreased physiological functions as growing older. This is called the aging phenomenon. Similarly, individual cells constituting a living body also undergo aging, during the course of which the cells change their traits, regress in terms of functions, and subsequently stop proliferation, eventually leading to cell death.

Conventionally, known is a method of evaluating an aging condition based on the length of telomere in chromosome obtained from a subject (for example, see Patent Document 1).

Patent Document 1: Japanese Unexamined Patent Application, Publication No. 2001-161364

### DISCLOSURE OF THE INVENTION

### Problems to be Solved by the Invention

However, evaluation results of aging can be more accurately determined as the number of options of markers capable of accurately evaluating an aging condition increases. This is because aging is a phenomenon involving various factors.

Accordingly, an object of the present invention is to provide a novel method of evaluating an aging condition, a method of presenting information, and a method of screening for a substance capable of improving or preventing an aging condition.

### Means for Solving the Problems

The present inventors found that the amount of extracellular vesicles including exosomes in serum and the amount of advanced glycation end products (AGEs) of surface proteins on extracellular vesicles are correlated with an aging condition. Then the present invention has been completed. Specifically, the present invention can provide the followings.
(1) A method of evaluating an aging condition of a subject, the method comprising the step of: comparing a value from the subject of either of the amount of extracellular vesicles including exosomes in serum, the amount of advanced glycation end products of surface proteins on the extracellular vesicles, or parameters based on the amounts with a correlation criterion, the correlation criterion with the aging condition being pre-determined.
(2) A method of presenting information about a subject, the method comparing the steps of: evaluating an aging condition of the subject in accordance with the method according to (1); and in a case where the aging condition is evaluated not to be in a target condition, presenting information registered in a database as an ingredient capable of shifting the values of the amounts or the parameters towards numerical ranges of the amounts or the parameters as determined based on the correlation criterion when the aging condition is in the target condition.
(3) The method according to (2), further comprising the steps of: re-evaluating the aging condition of the subject after the ingredient is ingested in accordance with the method according to claim 1; and updating the information based on the degree of improvement in the aging condition re-evaluated.
(4) A method of screening for a substance capable of improving or preventing an aging condition, the method comprising the step of: selecting a candidate substance based on a change in either of the amount of extracellular vesicles including exosomes in serum, the amount of advanced glycation end products of surface proteins on the extracellular vesicles, or parameters based on the amounts due to the candidate substance, a correlation criterion with the aging condition being pre-determined.

### Effects of the Invention

The present invention can provide a novel method of evaluating an aging condition, a method of presenting information, and a method of screening for a substance capable of improving or preventing an aging condition.

### BRIEF DESCRIPTION OF THE DRAWINGS

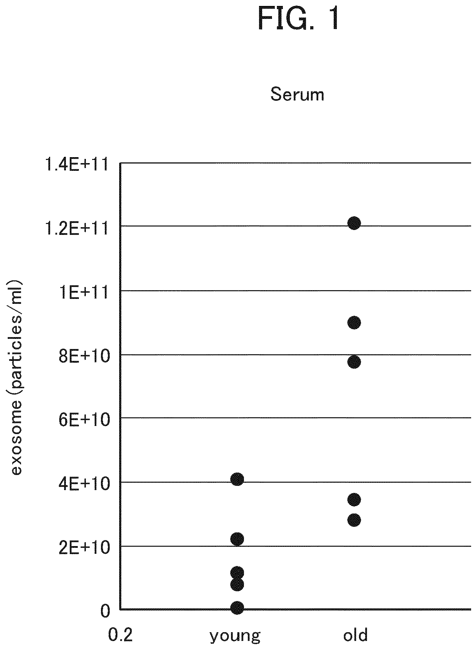

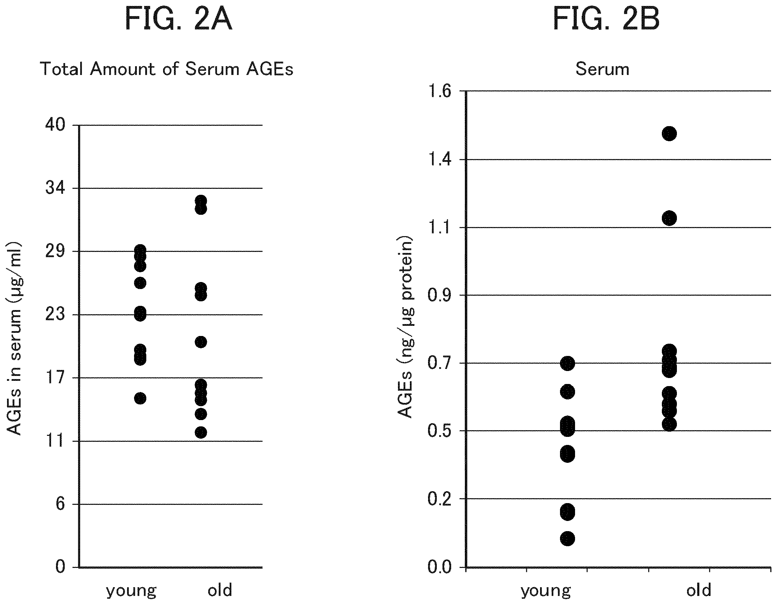

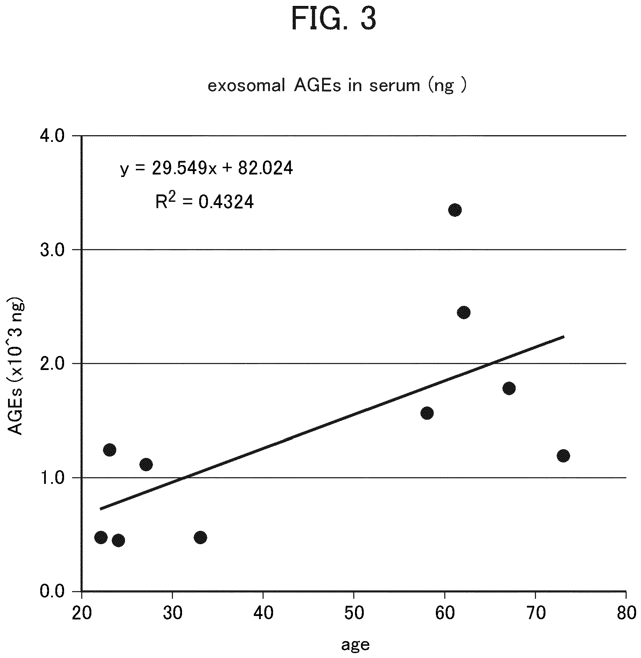

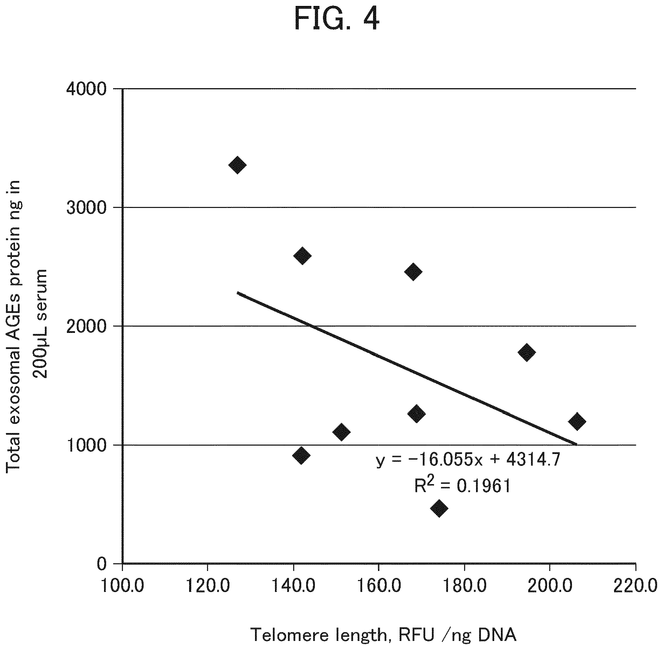

Fig. 1 shows a graph indicating the relationship between the ages of subjects and the amounts of extracellular vesicles including exosomes in serum. It is noted that "young" refers to a subject of actual age of 35 or younger, and "old" refers to a subject of actual age of 60 or older. Fig. 2 shows (a) a graph indicating the relationship between the ages of subjects and the total amount of advanced glycation end products in serum, and (b) a graph indicating the relationship between the ages of subjects and the amounts of advanced glycation end products of surface proteins on extracellular vesicles in serum. It is noted that "young" refers to a subject of actual age of 35 or younger, and "old" refers to a subject of actual age of 60 or older. Fig. 3 shows a graph indicating the relationship between the ages of subjects and the amounts of advanced glycation end products of surface proteins on extracellular vesicles in serum. It is noted that the horizontal axis represents the actual ages of subjects. Fig. 4 shows a graph indicating the relationship between the amounts of advanced glycation end products of surface proteins on extracellular vesicles in serum and the lengths of telomere.

### PREFERRED MODE FOR CARRYING OUT THE INVENTION

Below, the embodiments of the present invention will be described, but the present invention shall not be limited to these.

An embodiment of the present invention is a method of evaluating an aging condition of a subject, the method comprising the step of: comparing a value from the subject of either of the amount of extracellular vesicles including exosomes in serum, the amount of advanced glycation end products of surface proteins on the extracellular vesicles, or parameters based on the amounts with a correlation criterion, the correlation criterion with the aging condition being pre-determined. This enables objective evaluation of the aging condition. Further, the aging condition, which is conventionally perceived collectively, can be subcategorized based on the values of the above amounts or parameters. This may enable the aging condition of a subject to be improved more reliably. It is noted that the values of the above amounts or parameters are highly correlated with the aging condition, but not particularly highly correlated with an aging marker other than these (for example, the length of chromosomal telomere of a subject). For this reason, the above amounts or parameters are preferably used in combination with an aging marker other than these in view of the fact that the aging condition can be evaluated more accurately in terms of various aspects.

Another embodiment of the present invention is a method of presenting information about a subject, the method comprising the steps of: evaluating the aging condition of the subject in accordance with the above method; and in a case where the aging condition is evaluated not to be in a target condition, presenting information registered in a database as an ingredient capable of shifting the values of the amounts or the parameters towards numerical ranges of the amounts or the parameters as determined based on the correlation criterion when the aging condition is in the target condition. This enables information for improving the aging condition and the like be presented to the subject in a personalized form. It is noted that the term "information" as used herein refers to the name of an ingredient or an ingredient-containing formulation, the usage/dose of an ingredient/formulation, the way or degree of expected improvement in the aging condition, side effects, and the like.

In an embodiment, the method preferably further includes the steps of: re-evaluating the aging condition of the subject after the ingredient is ingested in accordance with the above method of evaluation; and updating the information based on the degree of improvement in the aging condition re-evaluated. This may enable information in the data base to be improved, leading to more reliable improvement in the aging condition, and the like.

There is no particular limitation for the timing of re-evaluation, but the timing may be, for example, a point where time has passed enough for expecting an improved aging condition after the ingredient is used (which may be included in the data base).

Another embodiment of the present invention is a method of screening for a substance capable of improving or preventing an aging condition, the method comprising the step of: selecting a candidate substance based on a change in either of the amount of extracellular vesicles including exosomes in serum, the amount of advanced glycation end products of surface proteins on the extracellular vesicles, or parameters based on the amounts due to the candidate substance, a correlation criterion with the aging condition being pre-determined. This may enable a substance capable of improving or preventing an aging condition to be screened with a high probability.

It is noted that in the screening according to the embodiments of the present invention, the probably of the presence of a subject capable of improving or preventing an aging condition may be increased in the group of candidate substances after the screening as compared with that in the group of candidate substances before the screening. A candidate substance after the screening, however, does not necessarily need to improve or prevent the aging condition. Therefore, it is preferred to further perform a step of determining if a screened candidate substance has an effect for improving or preventing the aging condition.

There is no particular limitation for the parameters as long as they are based on the amount of extracellular vesicles including exosomes in serum or the amount of advanced glycation end products of surface proteins on extracellular vesicles. The parameters may be the difference, sum, product, ratio, or a combination thereof of the amounts. Among these, the parameters may be the sum, product, or a combination thereof.

The subject may be any of a living body, an organ, a tissue, and a cell. The organ, tissue, and cell may be those taken from a living body, or those differentiated/regenerated from a stem cell.

Extracellular vesicles mediate communication between cells, and are involved in various processes such as immune response and blood coagulation. They are classified into exosomes, microvesicles, and apoptotic bodies according to their properties.

Among extracellular vesicles, exosomes has a particle size of 40 to 120 nm as measured with a nanoparticle multianalyzer qNano for measuring particle size distribution and particle diameter (available from Izon Science), and marker molecules thereof include CD9 and CD63. The presence of an exosome can be determined by detecting the presence of CD9 and CD63 with Western blotting.

The amount of extracellular vesicles including exosomes may be the number of extracellular vesicles, and measured by isolating exosomes from serum according to a conventional method and counting the number of particles included in a fraction thereof with a nanoparticle multianalyzer qNano (available from Izon Science) and the like for measuring particle size distribution and particle diameter.

Surface proteins on extracellular vesicles are proteins which are present on the lipid bilayer membranes of extracellular vesicles.

The amount of advanced glycation end products of surface proteins may be a proportion of advanced glycation end products in the surface proteins. Here, the amount of surface proteins may be determined by the BCA protein assay of exosomes isolated from serum. The amount of advanced glycation end products may be determined in exosomes isolated from serum according to a conventional method such as ELISA (OxiSelect™ Advanced Glycation End Product (AGE) Competitive ELISA kit (Cell Biolabs Inc.) or similar kits).

There is no particular limitation for the aging condition as long as it shows a correlation with the above amounts or parameters, but it can be a qualitative or quantative condition associated with aging. Specific examples of the aging condition include cellular senescence, ages of individual parts (for example, skin, blood vessel, muscle), and the manifested or potential severity of symptoms arising from the progress of aging (for example, myocardial infarction, cancer, dementia, cerebral infarction, arteriosclerosis, and the like).

The correlation criterion can determine an aging condition to be evaluated qualitatively or quantitatively based on the above amounts or parameters, and typically a correlation formula between the above amounts or parameters and the aging condition. An example of evaluation of an aging condition may be, for example, evaluation of whether the aging condition as determined from the values of the above amounts or parameters and the correlation criterion is advanced further than expected from the actual age of a subject.

Many of aging conditions can be correlated with not only the amount of extracellular vesicles including exosomes in serum and the amount of advanced glycation end products of surface proteins on the extracellular vesicles but also other phenomena (for example, the actual age). For this reason, a correlation criterion is preferably used in which influences due to at least one of events known to be correlated with aging conditions has been eliminated. This can suppress such influences due to the presence or absence or the degree of the above other events with regard to a subject, and enables more accurate evaluation of physical conditions based on the amount of extracellular vesicles including exosomes in serum, the amount of advanced glycation end products of surface proteins on the above extracellular vesicles, or the value of a parameter. Further, in the method of presenting information about a subject, more appropriate information can be presented by considering the presence or absence or the degree of the above other events with regard to a subject. For this reason, the presence or absence or the degree of the above other events with regard to a subject will preferably be inputted along with the amount of extracellular vesicles including exosomes in the above serum, the amount of advanced glycation end products of surface proteins on the above extracellular vesicles, or the parameters. Further, in the method of screening for a substance capable of improving or preventing an aging condition, the substance capable of improving or preventing an aging condition may be more easily obtained regardless of the above other events of a user.

### EXAMPLES

### 1. Clinical Specimens Used

Collection of peripheral blood was performed in accordance with the human genome research proposal approved by the Ethical Committee for Human Genome Research of Hiroshima University. With regard to blood, peripheral blood was obtained from 20 healthy volunteers having neither chronic diseases nor under-treatment diseases. The details of peripheral blood used for analysis in this example are as follows: 10 elderly persons (6 males and 4 females; the average of 66.5 years old, 58 to 75 years old) and 10 young persons (6 males and 4 females; the average of 26.2 years old, 22 to 33 years old).

### 2. Recovery and Storage of Serum

Peripheral blood was collected into a Venoject II vacuum blood collection tube (TERUMO cat. VP-AS106K50) containing a serum separator. The blood collection tube was incubated for 30 minutes at room temperature, and then centrifuged at 2330×g and room temperature for 10 minutes. The upper serum was transferred to a 2-mL tube, and further centrifuged at 12000×g and 4°C for 10 minutes. The supernatant was dispensed each about 0.3 mL into 1.5 mL tubes, and cryopreserved at -80°C.

### 3. Purification and Confirmation of Exosomes in Serum

Exosomes were purified from 200 µL of serum with a Total Exosome Isolation kit from serum (Invitrogen, cat. 4478360) according to the protocol of the kit, and suspended in 100 µL of 1×PBS(-) buffer. The number and size of the purified particles were measured with a qNano (Izon Science). Further, the presence of CD63 and CD9, which are marker proteins for exosomes, was checked in the purified particles by Western blotting. The relationship between the ages of subjects and the amounts (the number of particles) of extracellular vesicles including exosomes in serum is shown in Fig. 1.

### 4. Quantification of Proteins

Quantification of proteins in the exosome sample was also performed with a Micro BCA protein assay kit (Thermo Fisher Scientific, cat. 23235).

### 5. Quantification of Advanced Glycation End Products

Quantification of advanced glycation end products residing on the surfaces of exosomes in serum was performed with OxiSelect™ Advanced Glycation End Product according to the protocol of the kit after the exosome-containing sample obtained from the above section 3 was diluted with a 1×PBS(-) buffer to a concentration of 10 mg/ml. Fig. 2(b) shows the relationship between the ages of subjects and the amounts of advanced glycation end products of surface proteins on extracellular vesicles in serum. Fig. 3 shows the relationship between the ages of subjects and the amounts of advanced glycation end products of surface proteins on extracellular vesicles in serum.

Advanced glycation end products of exosomes were quantified by competitive inhibition ELISA (Enzyme Linked Immunosolvent Assay) using an advanced glycation end product-BSA with a known concentration of advanced glycation end products as a standard. Advanced glycation end product-BSA having synthesized advanced glycation end products bound to BSA was immobilized to a microplate. Then, a mouse anti-advanced glycation end product antibody was allowed to react with the immobilized advanced glycation end product-BSA in the presence of an advanced glycation end product-BSA standard diluted to a plurality of concentrations or a sample (serum obtained from the above section 2). An enzyme-labeled antimouse IgG antibody was allowed to react with the bound mouse anti-advanced glycation end product antibody, and a substrate was then added to measure the activity of the enzyme remained in the microplate. Based on the absorbance of the advanced glycation end product-BSA standard, the concentration of the total advanced glycation end products within the sample was computed. Fig. 2(a) shows the relationship between the ages of subjects and the total amounts of advanced glycation end products in serum.

### 6. Quantification of Telomere Length

DNA was extracted from cells obtained from healthy volunteers, and the length of telomere was determined according to the method described in Japanese Patent No. 5514401. Fig. 4 shows the relationship between the amounts of advanced glycation end products of surface proteins on extracellular vesicles in serum and the lengths of telomere.

As shown in Fig. 1, the amount of extracellular vesicles including exosomes in serum was found to increase as the age of a subject increased. Further, as shown in Fig. 2(b), the amount of advanced glycation end products of surface proteins on extracellular vesicles including exosomes in serum was found to increase as the age of a subject increased. As shown in Fig. 3, the amount of advanced glycation end products of surface proteins on extracellular vesicles including exosomes in serum was found to show a reasonable correlation with the age of a subject. In contrast, the total amount of advanced glycation end products in serum did not show correlation with the age of a subject, as shown in Fig. 2(a). As described above, the amount of extracellular vesicles including exosomes in serum and the amount of advanced glycation end products of surface proteins on extracellular vesicles including exosomes showed correlation with an aging condition correlated strongly with age. This means that an aging condition can be evaluated by comparing the values of the amounts with correlation formulas.

As shown in Fig. 4, the amount of advanced glycation end products of surface proteins on extracellular vesicles including exosomes in serum was also correlated with the length of telomere. Further, this correlation is somewhat weaker than that shown in Fig. 3, suggesting that the above amounts and the length of telomere may reflect partly different aspects of an aging condition. For this reason, an aging condition can be evaluated more accurately in terms of various aspects by using the above amounts or parameters in combination with an aging marker other than these.

## Claims

1. A method of evaluating an aging condition of a subject, the method comprising the step of:
comparing a value from the subject of either of the amount of extracellular vesicles including exosomes in serum, the amount of advanced glycation end products of surface proteins on the extracellular vesicles, or parameters based on the amounts with a correlation criterion, the correlation criterion with the aging condition being pre-determined.

2. A method of presenting information about a subject, the method comparing the steps of:
evaluating an aging condition of the subject in accordance with the method according to claim 1; and
in a case where the aging condition is evaluated not to be in a target condition, presenting information registered in a database as an ingredient capable of shifting the values of the amounts or the parameters towards numerical ranges of the amounts or the parameters as determined based on the correlation criterion when the aging condition is in the target condition.

3. The method according to claim 2, further comprising the steps of: re-evaluating the aging condition of the subject after the ingredient is ingested in accordance with the method according to claim 1; and
updating the information based on the degree of improvement in the aging condition re-evaluated.

4. A method of screening for a substance capable of improving or preventing an aging condition, the method comprising the step of:
selecting a candidate substance based on a change in either of the amount of extracellular vesicles including exosomes in serum, the amount of advanced glycation end products of surface proteins on the extracellular vesicles, or parameters based on the amounts due to the candidate substance, a correlation criterion with the aging condition being pre-determined.
